# EUROPÄISCHE PATENTSCHRIFT

(11) **EP 1 218 006 B1**
(45) Veröffentlichungstag und Bekanntmachung des Hinweises auf die Patenterteilung: **03.03.2010**
(21) Anmeldenummer: 00967789.9
(22) Anmeldetag: 26.09.2000
(51) Int. Cl.: A61K 31/4439, A61P 35/00, A61P 35/02, A61P 35/04, A61P 9/00

(54) **INDOLYL-3-GLYOXYLSÄURE-DERIVATE MIT THERAPEUTISCH WERTVOLLEN EIGENSCHAFTEN**
INDOLYL-3-GLYOXYLIC ACID DERIVATIVES COMPRISING THERAPEUTICALLY VALUABLE PROPERTIES
DERIVES D'ACIDE INDOLYL-3-GLYOXYLE A BONNES PROPRIETES THERAPEUTIQUES

(30) Priorität: 28.09.1999 DE 19946301
(43) Veröffentlichungstag der Anmeldung: 03.07.2002
(73) Patentinhaber: ZIOPHARM ONCOLOGY, INC., Charlestown MA 02129 (US)
(72) Erfinder: NICKEL, Bernd, 64367 Mühltal (DE); KLENNER, Thomas, 55218 Ingelheim (DE); BACHER, Gerald, 82110 Germering (DE); BECKERS, Thomas, 60596 Frankfurt (DE); EMIG, Peter, 63486 Bruchköbel (DE); ENGEL, Jürgen, 63755 Alzenau (DE); BRUYNEEL, Erik, B-8530 Harelbeke (BE); KAMP, Günter, 48149 Münster (DE); PETERS, Kirsten, 55128 Mainz (DE)
(74) Vertreter: Hock, Joachim
(86) Internationale Anmeldenummer: PCT/EP2000/009390
(87) Internationale Veröffentlichungsnummer: WO 2001/022954

(56) Entgegenhaltungen:
- WO-A-00/67802
- WO-A-99/46237
- WO-A-99/51224
- WO-A-99/55696
- DE-A- 19 636 150
- PATENT ABSTRACTS OF JAPAN vol. 2000, no. 12, 3. Januar 2001 (2001-01-03) & JP 2000 239252 A (MITSUBISHI CHEMICALS CORP.), 5. September 2000 (2000-09-05)

## Beschreibung

Die Erfindung betrifft die weitere vorteilhafte Ausgestaltung der deutschen Patentanmeldung Indol-3-glyoxylamide mit dem Aktenzeichen 19814 838.0.

Im Zusammenhang mit der Chemotherapie bei Tumorerkrankungen ergeben sich die größten Probleme durch das Auftreten der Arzneimittel-Resistenz zum einen und durch die schwerwiegende Nebenwirkungen dieser Mittel zum anderen.

Ferner ist es bekannt, daß viele Primärtumore nach Erreichen einer gewissen Größe frühzeitig über Blut- und Lymphbahnen zur Metastasenbildung neigen. Der fortschreitende Prozeß von Tumorinvasion und die Bildung von Metastasen ist die häufigste Todesursache der Krebspatienten.

Für die Erklärung dieser Ausbreitung gibt es verschiedene Ansatzpunkte u.a. verstärkte Angiogenese, erhöhter extrazelllulärer Matrixabbau, Tumorzellmigration und Modulation der Zelladhäsion. Diese Faktoren können auch zusammenwirken sind aber bis heute nur partiell aufgeklärt.

Die Metastasierung eines Tumors ist meistens durch schlechte Prognosen bei der Tumorbehandlung begleitet. Voraussetzung der Metastasierung ist die Loslösung von Zellen aus dem primären Tumor, die Wanderung der Zellen zu den Blutgefäßen, die Invasion in die Blutgefäße und die Invasion der Zellen aus den Blutgefäßen in andere Gewebe.
Von bestimmten Tumormitteln wie Tamoxoifen ist eine Hemmwirkung auf die Migration und Invasion von Krebszellen bekannt [ J Clin Endocrinol Metab 1995 jan;80(1):308-13]

Über die Hemmung der Tumorzellinvasion durch Verapamil wurde berichtet [Pigment Cell Res 1991 Dec;4(5-6):225-33.]

Der Einfluß von Melantonin auf invasive und metastatische Eigenschaften von MCF-7 menschlichen Brustkrebszellen wurde berichtet [Cancer res 1998 Oct1;58(19):4383-90]

In der veröffentlichten PCT Anmeldung WO 96/23506 wurde die Überwindung der Arzneimittel-Resistenz bei gewissen Tumorpharmaka als Folge der durch solche Tumormittel bewirkten Genamplifikation des Multi- Drug - Resistance Gens (MDR-Gen) nachgewiesen.

Tumormittel wie Vincristin und Taxol weisen ferner eine nicht unerhebliche Neurotoxizität auf, die sich bei der Chemotherpie als nachteilig erweist.

Aufgabe der Erfindung ist es nun, den Einsatzbereich von N -substituierte Indol-3-glyoxylamide zu erweitern und somit den verfügbaren Arzneischatz zu bereichern.
Damit soll die Möglichkeit einer niedrigeren, länger anhaLtenden und besser verträglichen Medikation für die in der deutschen Patentanmeldung 19814 838.0. beschriebene Stoffklasse mit Antitumorwirkung eröffnet werden. Insbesondere soll die nachteilige Resistenzentwicklung, wie sie von vielen Antitumormitteln bekannt ist, umgangen werden.
Außerdem soll einer Entwicklung und Ausbreitung des Tumors durch Metastasen entgegengewirkt werden.

Da nach neueren Erkenntnissen für das Tumorwachstum und die Entwicklung von Metastasen offenbar auch die Angiogenese verantwortlich ist, stellt die Eigenschaft der Angiogenesehemmung einen weiteres vorteilhaftes Arzneimittelpotential zum Beispiel in der Krebstherapie dar.

Die mit den N -substituierten Indol-3-glyoxylamiden erzielte Wirkungsverstärkung soll den Arzneimittelverbrauch in der Tumortherapie effektiver gestalten. Darüber hinaus sollte es möglich sein, die Behandlungsdauer zu verkürzen und auf therapieresistente Fälle auszudehnen. Ferner sollen Rezidive und Metastasen eingeschränkt bzw. verhindert und somit die Überlebensdauer der Patienten zusätzlich erhöht werden. Ziel ist es, Medikamente zu entwickeln, die in den Metastasierungsprozeß eingreifen können.

Es wurde überraschend gefunden, daß die in der deutschen Patentanmeldung 19814 838.0 beschriebenen N -substituierten Indol-3-gloxylamide der nachfolgend angegebenen allgemeinen Formel 1, die zur Behandlung von Tumorerkrankungen geeignet sind, weitere für die Tumorbehandlung solche vorteilhaften Eigenschaften besitzen, die ihr Einsatzgebiet erweitern können.

Gegenstand der Erfindung ist die Verwendung von N -substituierten Indol-3-gloxylamiden gemäß Formel 1 zur Tumorbehandlung bei Arzneimittelresistenz 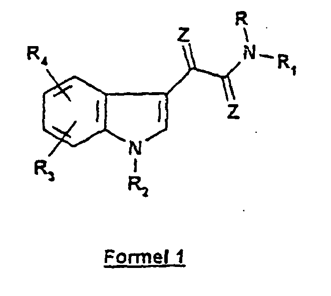 wobei die Reste R, R₁, R₂, R₃, R₄ und Z folgende Bedeutung haben:
- R=: Wasserstoff
- R₁: ein Pyridin-Gerüst der Formel 2 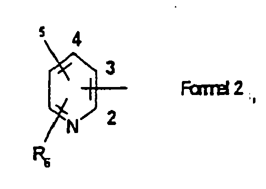 wobei das Pyridin-Gerüst wahlweise an den Ringkohlenstoff Atomen 2, 3 und 4 gebunden ist und mit den Substituenten R₅ und R₆ substituiert sein kann. Die Reste R₅ und R₆ können gleich oder verschieden sein und die Bedeutung (C₁ -C₆)-Alkyl sowie die Bedeutung (C₃ -C₇)-Cycloalkyl, (C₁ -C₆)-Alkoxy, Nitro, Amino, Hydroxy, Halogen und Trifluormethyl besitzen und ferner den Ethoxycarbonylamino-Rest sowie die Gruppe Carboxyalkyloxy darstellen, bei dem die Alkylgruppe über 1-4 C-Atome verfügen kann.
- R₂: die (C₁ -C₆)-Alkyl-Gruppe bedeutet, wobei die Alkylgruppe ein- oder mehrfach durch Halogen und Phenyl substituiert ist, das seinerseits ein- oder mehrfach durch Halogen, (C₁ -C₆)-Alkyl, (C₃ - C₇)-Cycloalkyl, Carboxylgruppen
mit C₁ -C₆-Alkanolen veresterten Carboxylgruppen, Trifluormethylgruppen, Hydroxylgruppen, Methoxygruppen, Ethoxygruppen oder Benzyloxygruppen
substituiert sein kann. Die für R₂ geltende (C₁-C₆)-Alkyl-Gruppe kann ferner durch die 2-Chinolylgruppe und das 2-,3- und 4-Pyridyl-Gerüst substituiert sein, die beide
jeweils ein- oder mehrfach durch Halogen, (C₁ -C₄)-Alkylgruppen oder (C₁ - C₄)-Alkoxy-gruppen substituiert sein können.
- R₃ und R₄: können gleich oder verschieden sein und Wasserstoff, (C₁ -C₆)-Alkyl, (C₃ -C₇)-Cycloalkyl, (C₁ -C₆)-Alkanoyl, (C₁ -C₆)-Alkoxy, Halogen und Benzyloxy bedeuten. Weiterhin können R₃ und R₄ die Nitrogruppe, die Aminogruppe, die (C₁ -C₄)-mono- oder dialkylsubstituierte Aminogruppe, und die (C₁ -C₆)- Alkoxy-carbonylamino-Funktion oder (C₁ -C₆)-Alkoxycarbonylamino-(C₁ -C₆)- alkyl-Funktion bedeuten.
- Z: steht für O und S

Unter der Bezeichnung Alkyl-, Alkanol-, Alkoxy- oder Alkylaminogruppe sind für die Reste R, R₁, R₂, R₃, R₄, R₅, R₆, regelmäßig sowohl "geradkettige" als auch "verzweigte" Alkylgruppen zu verstehen, wobei "geradkettige Alkylgruppen" beispielsweise Reste wie Methyl, Ethyl, n-Propyl, n-Butyl, n-Pentyl, n-Hexyl bedeuten können und "verzweigte Alkylgruppen" beispielsweise. Reste wie Isopropyl oder tert.-Butyl bezeichnen. Unter "Cycloalkyl" sind Reste wie beispielsweise Cyclopropyl, Cyclobutyl, Cyclopentyl, Cyclohexyl oder Cycloheptyl zu verstehen.
Die Bezeichnung "Halogen" steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy dar.

Die Verbindungen können auch als Säureadditionssalze eingesetzt werden, beispielsweise als Salze von Mineralsäuren, wie beispielsweise Salzsäure, Schwefelsäure, Phosphorsäure, Salze von organischen Säuren, wie beispielsweise Essigsäure, Milchsäure, Malonsäure, Maleinsäure, Fumarsäure, Gluconsäure, Glucuronsäure, Zitronensäure, Embonsäure, Methansulfonsäure, Trifluoressigsäure, Bernsteinsäure und 2-Hydroxy-ethansulfonsäure.

Sowohl die Verbindungen der Formel 1 als auch deren Salze sind biologisch aktiv. Die Verbindungen der Formel 1 können in freier Form oder als Salze mit physiologisch verträglichen Säuren verabreicht werden.
Die Applikation kann peroral, parenteral, intravenös, transdermal oder inhalativ vorgenommen werden.

Die Verbindungen der Formel 1 oder deren Salze können mit physiologisch verträglichen anorganischen oder organischen Säuren und gegebenenfalls pharmazeutisch verwendbaren Träger- und/oder Verdünnungs - bzw. Hilfsstoffen kombiniert werden.

Als Applikationsformen eignen sich beispielsweise Tabletten, Dragees, Kapseln, Lösungen zur Infusion oder Ampullen, Suppositorien, Pflaster, inhalativ einsetzbare Pulver-zubereitungen, Suspensionen ,Cremes und Salben.
Die Herstellungsverfahren für die Substanzen können den Beispielen aus dem Deutschen Patent DE 196 36 150 A1 entnommen werden.
Die gefundenen, therapeutisch wertvollen Eigenschaften, beziehen sich im einzelnen auf die nachfolgenden Vorteile:
- Es wurde keine Resistenzentwicklung nachgewiesen
- Es wurden Parameter nachgewiesen, die charakteristisch sind für die Hemmung der Metastasenbildung (Migration)
- Es wurden Parameter gefunden, welche die Hemmung der Gefäßneubildung belegen (Angiogenese)
- In verschiedenen Modellen konnte mit den N -substituierten Indol-3-gloxylamiden gemäß Formel 1 im Gegensatz zu den meisten Antitumorpräparaten keine Neurotoxizität gefunden werden

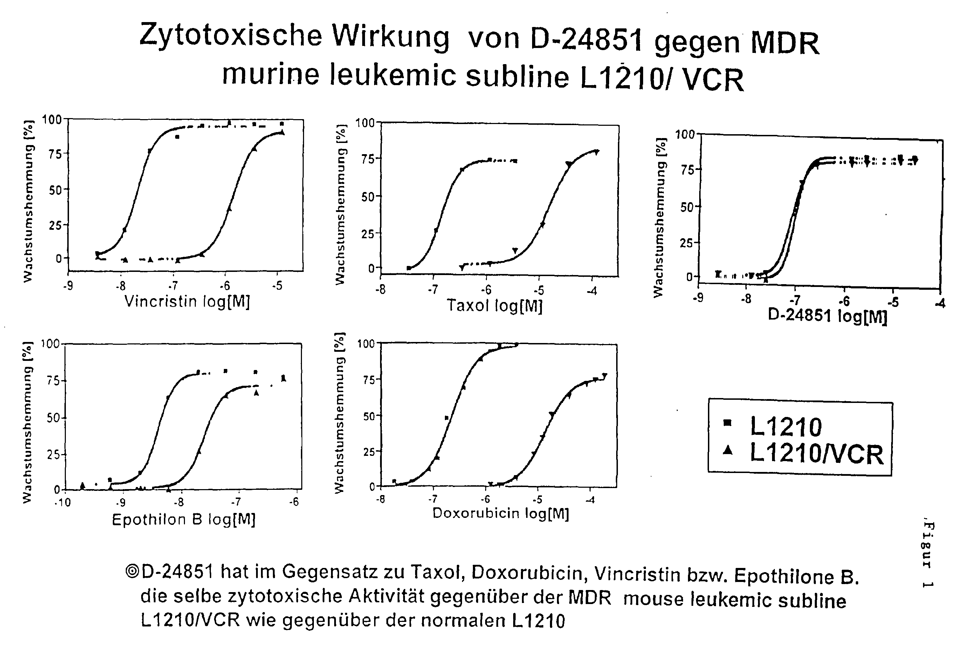

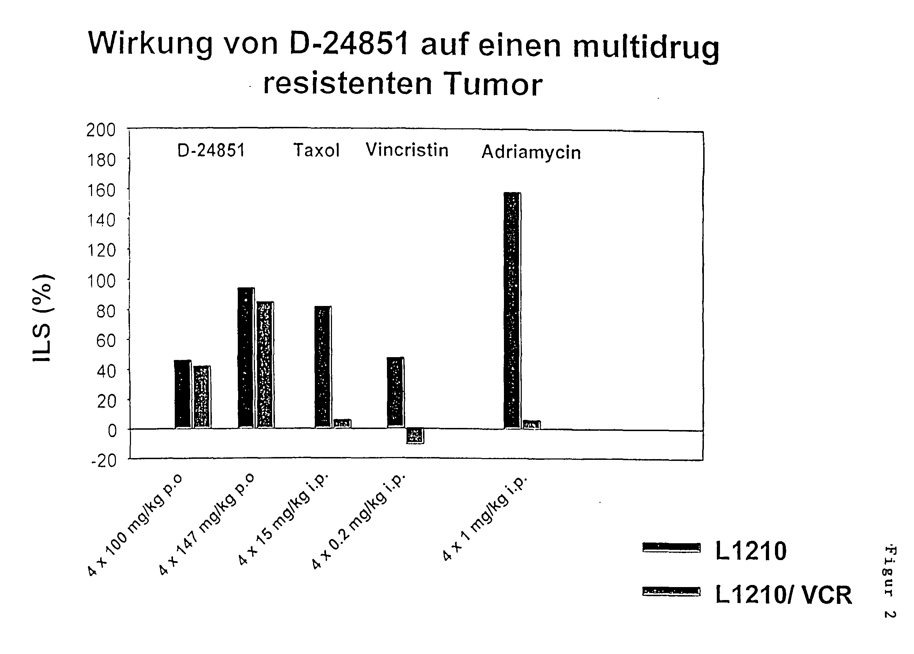

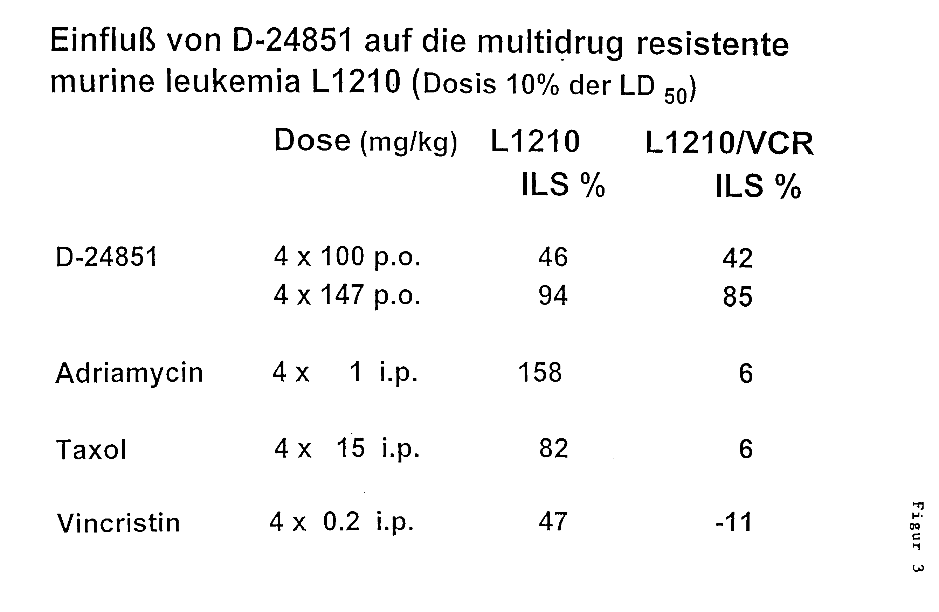

Die nicht vorhandene Resistenzentwicklung wird an nachfolgenden pharmakologischen Modellen bzw. Zellkulturen bewiesen :
1. Die cytotoxische Aktivität von D-24851 (siehe Anspruch 4) auf die MDR (multidrug resistente) Leukämiezellinie der Maus L 1210/VCR wird in vivo und in vitro nicht beeinflußt. Siehe Figur 1, 2 und 3.
   D-24851 ( siehe Anspruch 4) hat eine ungeänderte zytotoxische Aktivität gegen die multidrug resistente Mäuseleukämiezell- Sublinie L1210/VCR im Gegensatz zu Taxol, Doxirubicin, Vincristin oder Epotholon B.

### Versuchsdurchführung:

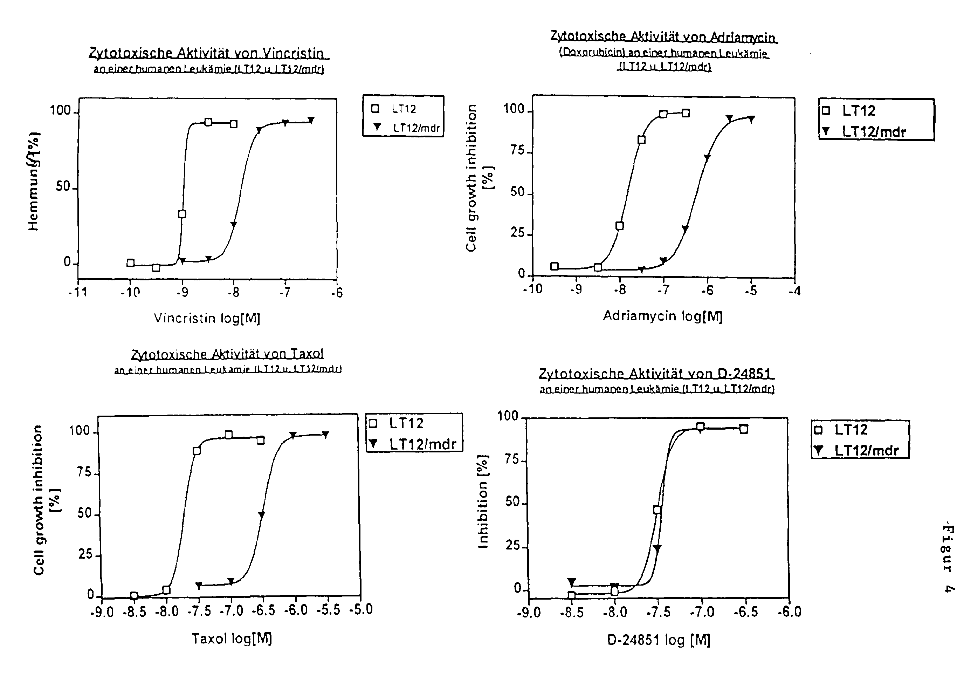

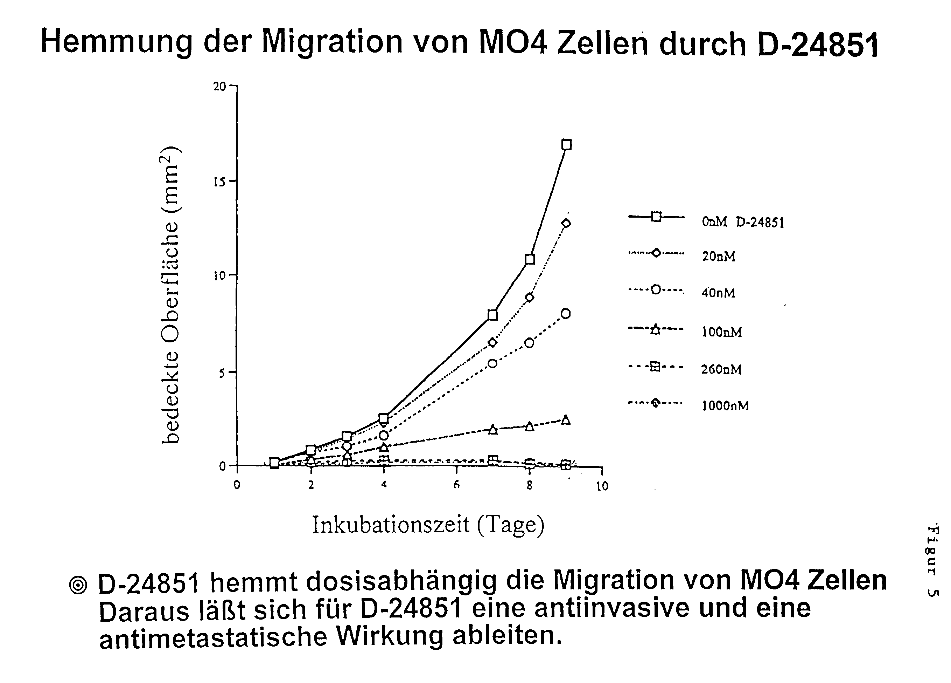

Die Mausleukämie Zellinien L 1210 wurde adaptiert an Vincristin. Die nicht adaptierten (L1210) und die adaptierten (L1210/VCR) Zellen wurden zytostatischen Mitteln ausgesetzt und das Zellwachstum, welches durch die metabolische Aktivität bestimmt wurde, wurde bestimmt (XTT Test).
Die Kurven, welche die XTT Datenpunkte verbinden, wurden berechnet unter Verwendung eines nichtlinearen Regressionsprogrammes.
Diese Versuchsergebnisse bestätigten sich in vitro auch an der humanen resistenten LT12/MDR Zelllinie siehe Figur 4.
2. Der Nachweis einer fehlenden Metastasenbildung wurde erbracht über die Migrationshemmung von M04 Zellen. Siehe Figur 5.
   D-24 851 (siehe Anspruch 4) hemmt dosis abhängig die Migration von MO4 Zellen. Daraus läßt sich für D-24851 eine antiinvasive und eine antimetastatische Wirkung ableiten.
   In vitro kann die Migrationsfähigkeit von MO4 Zellen gemessen werden, indem Zellen in der Mitte einer Zellkulturschale ausgesäht werden und die Wanderung mittels Radius bzw. die abgedeckte Fläche der Zellen nach verschiedenen Tagen mit und ohne D-24851 bestimmt wird. Figur 4 zeigt, daß die Migration der Zellen mit steigender D-24851 Konzentration abnimmt.

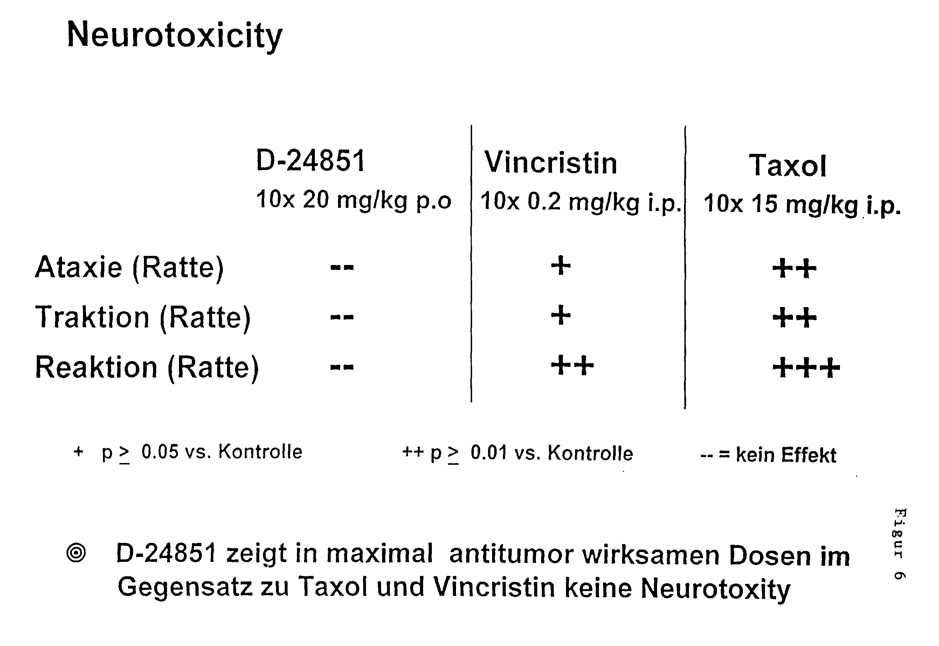

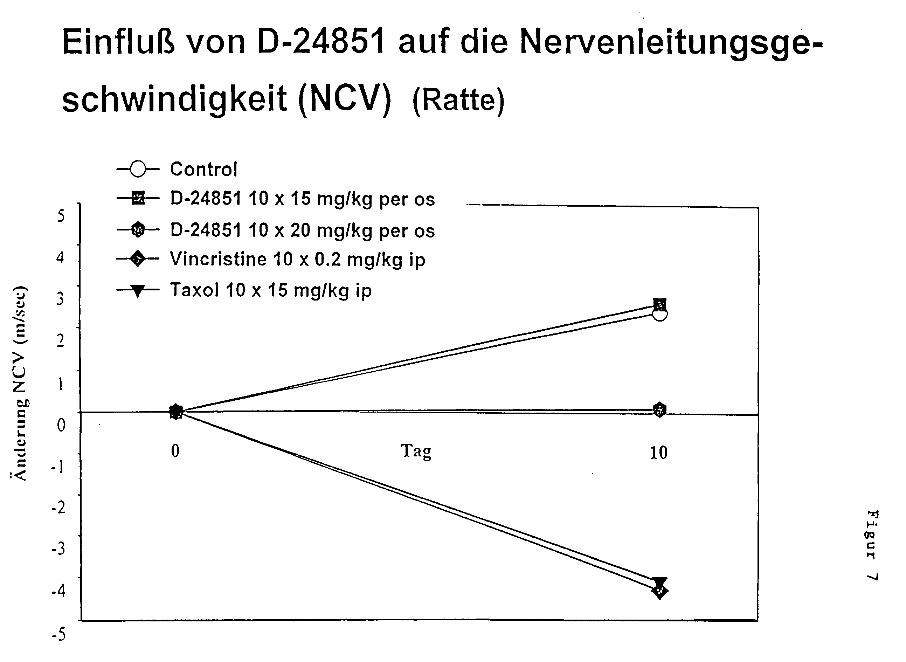

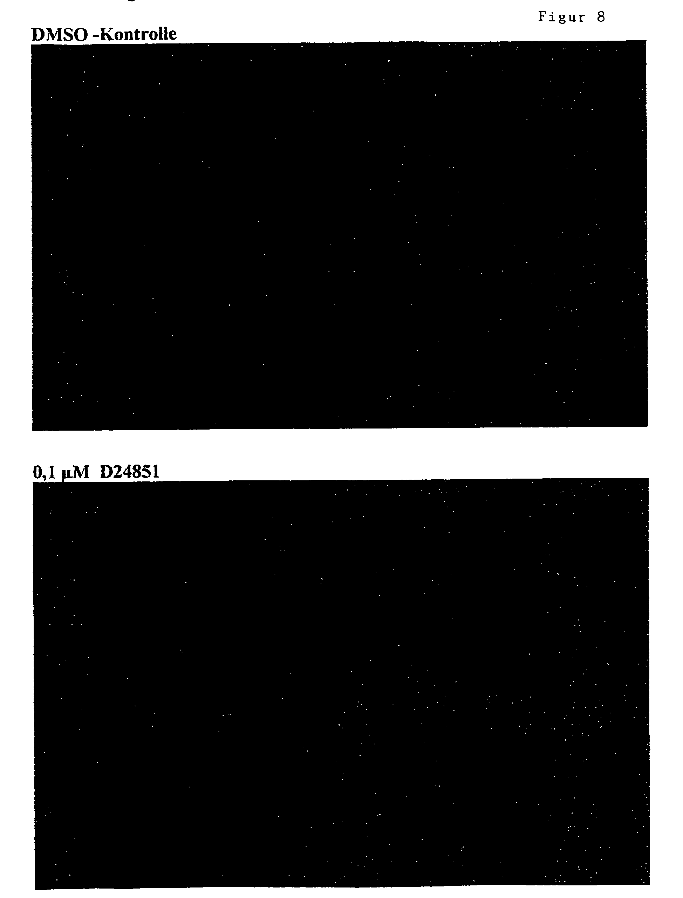

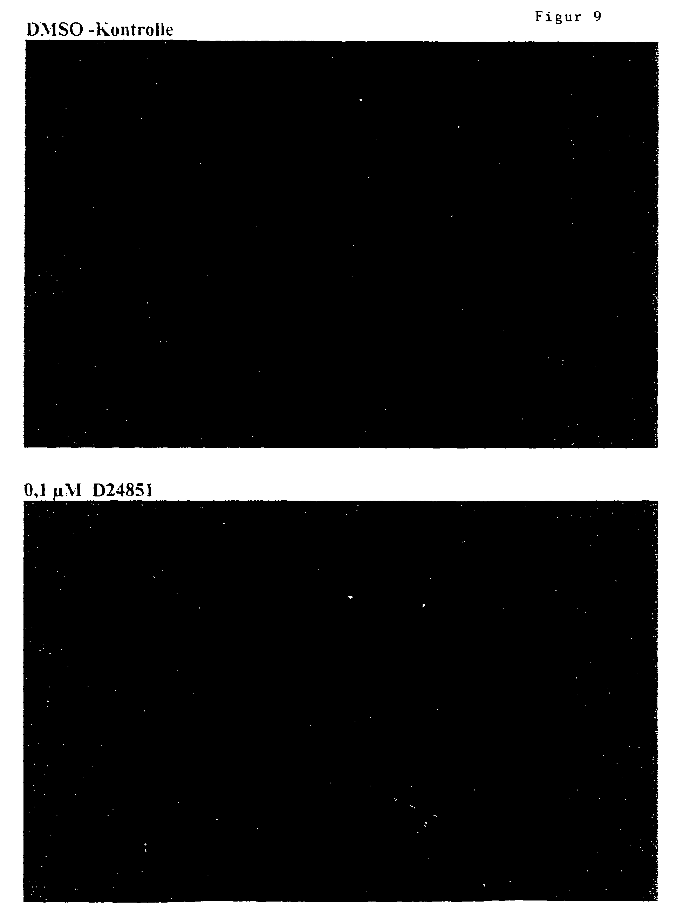

Um zu testen, ob D-24851 auch antiinvasiv wirkt, wurde die Invasion von MO4 Fibrosarcomzellen in Hühnerherz untersucht. Auch hier zeigt sich, daß bei Konzentration von 260 und 1000 nM die Invasion vollständig gehemmt wird, während bei geringeren Konzentrationen, die Invasionsfähigkeit der M04 Zellen zunimmt. Auf Grund dieser Befunde, zeigt sich, daß D-24851 sowohl die Migration als auch die Invasion von Tumorzellen hemmt und dadurch ein starkes antimetastastisches Potential besitzt.
3. Aus Vergleichsversuchen der erfindungsgemäß verwendeten Verbindung D-24851(siehe Anspruch 4) mit Vincristin und Taxol an Ratten, wobei Ataxie, Traktion und
   Reaktion bewertet wurden (siehe Figur 6), geht hervor, daß diese Verbindung im Gegensatz zu Taxol und Vincristin keinen neurotoxischen Effekt zeigt.
   D-24851 hat ferner im Vergleich zu Taxol und Vincristin keinen negativen Einfluß auf die Nervenleitungsgeschwindigkeit siehe Figur 7.
   Dies bestätigt, daß D- 24851 aufgrund der fehlenden Neurotoxicität über deutlich geringere Nebenwirkungen verfügt als andere Chemotherapeutika.
4. Aus weiteren Untersuchungen gem. Figur 8 und 9 ist ersichtlich, daß die Verbindung D-24851 (siehe Anspruch 4) über ein Potential als Angiogenesehemmer verfügt. Angiogesehemmstoffe sind infolge des physiologischen Zusammenhanges zum Tumorwachstum zugleich auch Mittel zur Hemmung des Tumorwachstums, indem die Bildung von neuen Blutgefäßen, welche den Tumor ernähren sollen, gehemmt wird.
   D-24851 ruft in vitro in einem Antiangiogenese - Modell an Endothelzellen eine komplette Hemmung der Gefäßbildung hervor, die nicht auf einem zytotoxischen Effekt beruht.
   In Figur 8 ist zu sehen, daß D-24851 bestehende Zell-Zellkontakte durch 0,1 µMol/L D 24851 nahezu vollständig auflöst (siehe Vitalfärbung). Normalerweise halten die Zellen zumindest partiell Kontakt. Die Zellmigration ist deutlich reduziert, viele Zellen sind abgerundet.
   Die Letalfärbung im Monolayer vor Angiogenese-Induktion zeigte mit D-24851 keinen erhöhte Zellmortalität. Auch in den ersten 22 Stunden nach Induktion war im Vergleich zur Kontrolle noch keine erhöhte Zellmortalität erkennbar.
   (siehe Letalfärbung in Figur 9. weiße Punkte)

Die Zellen stammten aus humaner Nabelschnurvene (arterielle Funktion). Sie wurden in dritter und vierter Passage für die Untersuchung eingesetzt. Die Angiogenese wird durch einen natürlichen Stimulus ausgelöst. Primärer Auslöser der endothelialen Migration ist ein Protein, welches in vaskularisierendem Gewebe verstärkt exprimiert wird. Die Substanzen werden dem Kulturmedium kurz vor der Angiogenese -Induktion zugesetzt.

Die Konzentration für die antiangiogenetische Wirkung von D-24851 liegt deutlich unterhalb der Konzentration für die zytotoxische Aktivität. Es ist dadurch möglich die beiden Wirkqualitäten ( zytotoxische Aktivität und antiangiogenetische Wirkung) von einander zu trennen.

Ohne mit der nach folgenden Angabe den Umfang der Erfindung einzuschränken zu wollen ist zu sagen, daß Dosierungen ab etwa 20 mg bis zu 500 mg täglich oral möglich sind.

Bei intravenöser Gabe als Injektion oder als Infusion können bis zu 250 mg/Tag oder mehr je nach Körpergewicht des Patienten und individueller Verträglichkeit verabreicht werden.

Infolge der fehlenden Resistenzentwicklung und Unterdrückung der Metastasierung ist eine hohe Effektivität und breiter Einsatz der Mittel zu auch bei tumorrefraktären Patienten zu erwarten.

Der antiangiogenese Effekt ist geeignet die Ausbreitung des Tumors zusätzlich zu unterdrücken.

Die N-substituierten Indol-3-gloxylamide gemäß Formel 1 können bei weiteren Erkrankungen bei denen funktionell ein Angiogense-Hemmeffekt erwüscht ist.(z.B. Wundheilung), eingesetzt werden.

Ferner ist Gegenstand der Erfindung auch die fixe oder frei Kombination der N-substituierten Indol-3-gloxylamide gemäß Formel 1 mit an sich bekannte Antitumormitteln, sowie der Ersatz von infolge Resistenzentwicklung unwirksam gewordener Antitumormittel durch N - substituierte Indol-3-gloxylamide gemäß Formel 1.

## Patentansprüche

1. N-substituierte Indol-3-glyoxylamide der folgenden Formel 1 bzw. physiologisch verträgliche Additionssalze davon zur Verwendung in der Behandlung von Arzneimittel-resistenten Tumoren 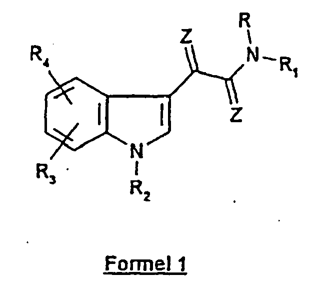 wobei die Reste R, R₁, R₂, R₃, R₄ und Z folgende Bedeutung haben:
R Wasserstoff,
R₁ ein Pyridingerüst der Formel 2 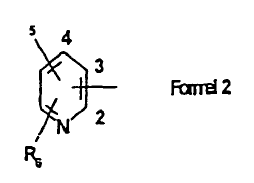 worin das Pyridin-Gerüst an den Ringkohlenstoffatomen 2, 3 oder 4 gebunden ist und wahlweise mit den Substituenten R₅ und R₆ substitusubstituiert sein kann, wobei die Reste R₅ und R₆ gleich oder verschieden sein können und die Bedeutung (C₁-C₆)-Alkyl, (C₃-C₇)-Cycloalkyl, (C₁-C₆)-Alkoxy, Nitro, Amino, Hydroxy, Halogen, Trifluormethyl, Ethoxycarbonylamino und Carboxyalkyloxy, bei dem die Alkylgruppe über 1-4 C-Atome verfügen kann, besitzen,
R₂ die (C₁-C₆)-Alkyl-Gruppe bedeutet, wobei die Alkylgruppe ein- oder mehrfach durch Halogen und Phenyl substituiert ist, das seinerseits ein- oder mehrfach durch Halogen, (C₁-C₆)-Alkyl, (C₃-C₇)-Cycloalkyl, ei- ne Carboxylgruppe, eine mit einem C₁-C₆-Alkanol veresterte Carboxyl- gruppe, eine Trifluormethylgruppe, eine Hydroxylgruppe, eine Methoxy- gruppe, eine Ethoxygruppe, eine Benzyloxygruppe, eine 2- Chinolylgruppe oder eine 2-, 3- oder 4-Pyridylgruppe substituiert ist, wobei die 2-Chinolyl- und 2-, 3- oder 4-Pyridylgruppe jeweils ein- oder mehrfach durch Halogen, eine (C₁-C₄)-Alkylgruppe oder eine (C₁-C₄)- Alkoxygruppe substituiert sein können,
R₃ und R₄ gleich oder verschieden sein können und Wasserstoff, (C₁-C₆)-Alkyl, (C₃-C₇)-Cycloalkyl, (C₁-C₆)-Alkanoyl, (C₁-C₆)-Alkoxy, Halogen, Benzyl- oxy, eine Nitrogruppe, eine Aminogruppe, eine (C₁-C₄)-mono- oder dialkylsubstituierte Aminogruppe, eine (C₁-C₆)-Alkoxy-carbonylamino- Gruppe und eine (C₁-C₆)-Alkoxycarbonylamino-(C₁-C₆)-alkylgruppe be- deuten, und
Z O oder S ist.

2. Verbindung gemäß Anspruch 1, wobei R₁ 4-Pyridyl ist, R₃ und R₄ Wasserstoff sind, und Z Sauerstoff ist.

3. Verbindung gemäß Anspruch 1 oder 2, wobei das Säureadditionssalz ein Salz einer Mineralsäure oder ein Salz einer organischen Säure ist.

4. Verbindung gemäß Anspruch 3, wobei die Mineralsäure aus Salzsäure, Schwefelsäure und Phosphorsäure ausgewählt ist, und die organische Säure aus Essigsäure, Milchsäure, Malonsäure, Maleinsäure, Fumarsäure, Gluconsäure, Glucuronsäure, Zitronensäure, Embonsäure, Methansulfonsäure, Trifluoressigsäure, Bernsteinsäure und 2-Hydroxyethansulfonsäure ausgewählt ist.

5. Verbindung gemäß einem der Ansprüche 1 bis 4, wobei das mindestens eine N-substituierte Indol-3-glyoxylamid mit einem pharmazeutisch verwendbaren Träger, Verdünnungsmittel oder Hilfsstoff kombiniert ist.

6. Verbindung gemäß einem der Ansprüche 1 bis 5, wobei das mindestens eine N-substituierte Indol-3-glyoxylamid als Tablette, Dragées, Kapsel, Lösung zur Infusion, Ampulle, Suppositorium, Pflaster, inhalativ einsetzbare Pulverzubereitung, Suspension, Creme oder Salbe formuliert ist.

7. Verbindung gemäß einem der Ansprüche 1, bis 6, wobei der Arzneimittelresistente Tumor gegenüber mindestens einem Antitumormittel, ausgewählt aus Taxol, Doxirubizin, Vincristin und Epothilon B, resistent ist.

8. Verbindung gemäß einem der Ansprüche 1 bis 7, wobei das eine oder die mehreren N-substituierten Indol-3-glyoxylamide in fixer oder freier Kombination mit einem bekannten Antitumormittel oder als Ersatz für aufgrund der Resistenzbildung nicht mehr wirksamer Antitumormittel verwendet wird.

9. Verbindung gemäß Anspruch 8, wobei das Antitumormittel, das in Kombination mit dem einen oder mehreren N-substituierten Indol-3-glyoxylamiden verwendet wird, aus Taxol, Doxirubicin, Vincristin und Epothilon B ausgewählt ist.

10. Verbindung gemäß Anspruch 8, wobei das aufgrund der Resistenzbildung nicht mehr wirksame Antitumormittel aus Taxol, Doxirubicin, Vincristin und Epothilon B ausgewählt ist.

11. Verbindung gemäß einem der Ansprüche 1 bis 10, wobei das N-substituierte Indol-3-glyoxylamid aus N-(Pyridin-4-yl)-[1-(4-fluorbenzyl)-indol-3-yl]-glyoxylamid, N-(Pyridin-4-yl)-(1-benzylindol-3-yl)-glyoxylamid, N-(Pyridin-4-yl)[1-(4-chlorbenzyl)-indol-3-yl]-glyoxylamid oder einem physiologisch verträglichen Säureadditionssalz davon ausgewählt ist.

## Claims

1. N-substituted indol-3-glyoxylamides of the following Formula I or physiologically tolerable acid addition salts thereof for use in treating drug-resistant tumors 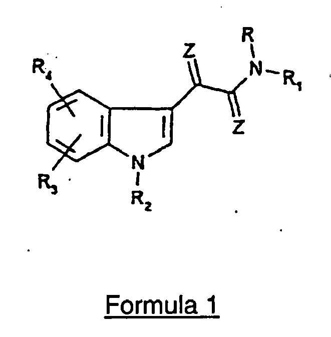 wherein the residues R, R₁, R₂, R₃, R₄ and Z have the following meanings:
R hydrogen;
R₁ a pyridine structure of Formula 2 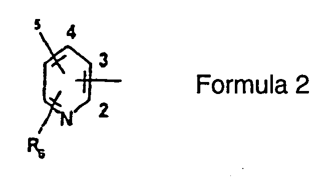 where the pyridine moiety is bonded to the ring carbon atoms 2, 3, or 4 and is optionally substituted by substituents R₅ and R₆, wherein R₅ and R₆ can be identical or different and represent (C₁-C₆)-alkyl, (C₃-C₇)-cycloalkyl, (C₁-C₆)-alkoxy, nitro, amino, hydroxyl, halogen, trifluoro-methyl, ethoxycarbonylamino and carboxyalkyloxy in which the alkyl group can have 1-4 atoms;
R₂ represents the (C₁-C₆)-alkyl group, where the alkyl group has one or more substituents selected from halogen and phenyl, which is optionally substituted by one or more substituents se- lected from halogen, (C₁-C₆)-alkyl, (C₃-C₇)-cycloalkyl, a carboxyl group, a carboxyl group esterified with a C₁-C₆-alkanol, a trifluoromethyl group, a hydroxyl group, a methoxy group, an ethoxy group, a benzyloxy group, a 2-quinolyl group or a 2-, 3- or 4-pyridyl group, wherein the 2-quinolyl and 2-, 3-, or 4-pyridyl groups can both in each case be mono- or polysubstituted by halogen, (C₁-C₄)-alkyl group or (C₁-C₄)-alkoxy;
R₃ and R₄ can be identical or different and are independently selected from hydrogen, (C₁-C₆)-alkyl, (C₃-C₇)-cycloalkyl, (C₁-C₆)- alkanoyl, (C₁-C₆)-alkoxy, halogen, benzyloxy, a nitro group, an amino group, a (C₁-C₄)-mono or dialkyl-substituted amino group, a (C₁-C₆)-alkoxycarbonylamino group, and a (C₁-C₆)- alkoxycarbonylamino-(C₁-C₆)-alkyl group; and
Z is O or S.

2. The compound according to claim 1, wherein R₁ is 4-pyridyl; R₃ and R₄ are hydrogen; and Z is oxygen.

3. The compound according to claim 1 or 2, wherein the acid addition salt is a salt of a mineral acid or a salt of an organic acid.

4. The compound according to claim 3, wherein the salt of the mineral acid is selected from hydrochloric acid, sulfuric acid, and phosphoric acid, and the salts or organic acids are selected from acetic acid, lactic acid, malonic acid, maleic acid, fumaric acid, gluconic acid, glucuronic acit, citric acid, embonic acid, methanesulfonic acid, trifluoroacetic acid, succinic acid, and 2-hydroxyethanesulfonic acid.

5. The compound according to anyone of claims 1 to 4, wherein the at least one N-substituted indol-3-glyoxylamide is combined with a pharmaceutically utilizable vehicle, diluent, or excipient.

6. The compound according to anyone of claims 1 to 5, wherein the at least one N-substituted indol-3-glyoxylamide is formulated as a tablet, coated tablet, capsule, solution for infusion, ampoule, suppository, patch, powder preparation suitable for inhalation, suspension, cream or ointment.

7. The compound according to anyone of claims 1 to 6, wherein the drug-resistant tumor is resistant to at least one antitumor drug selected from taxol, doxorubicin, vincristine, and epothilone B.

8. The compound according to anyone of claims 1 to 7, wherein the one or more N-substituted indol-3-glyoxylamides are used in fixed or free combination with a known antitumor agent, or as a replacement for tumor agents being not anymore efficient due to formation of resistance.

9. The compound according to claim 8, wherein the antitumor agent used in combination with the one or more N-substituted indol-3-glyoxylamides is selected from taxol, doxorubicin, vincristine, and epothilone B.

10. The compound according to claim 8, wherein the tumor agent being not anymore efficient due to formation of resistance is selected from taxol, doxorubicin, vincristine, and epothilone B.

11. The compound according to anyone of claims 1 to 10, wherein the N-substituted indol-3-glyoxylamide is selected from N-(pyridin-4-yl)-[1-(4-fluorobenzyl)indol-3-yl] glyoxylamide, N-(pyridin-4-yl)-(benzylindol-3-yl) glyoxylamide; N-(pyridin-4-yl)-[1-(4-chlorobenzyl)indol-3-yl] glyoxylamide; or a physiologically tolerable acid-addition salt thereof.

## Revendications

1. Indol-3-glyoxylamides N-substitués de la formule 1 suivante et respectivement, sels d'addition physiologiquement compatibles de ceux-ci, à utiliser dans le traitement de tumeurs résistant aux médicaments 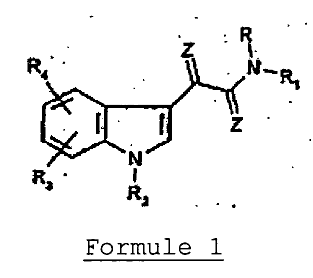 où les restes R, R₁, R₂, R₃, R₄ et Z ont la signification suivante :
R hydrogène,
R₁ un squelette pyridine de la formule 2 : 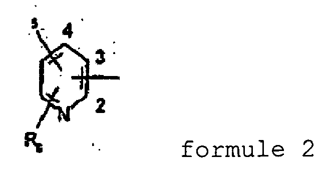 où le squelette pyridine est relié sur les atomes de carbone cycliques 2, 3 ou 4 et peut être substitué avec les substituants R₅ et R₆, où les restes R₅ et R₆ peuvent être identiques ou différents et possèdent la signification alkyle en C₁-C₆, cycloalkyle en C₃-C₇, alcoxy en C₁-C₆, nitro, amino, hydroxy, halogène, trifluorométhyle, éthoxycarbonylamino et carboxyalcoxy dans lequel le radical alkyle peut présenter 1-4 atomes C,
R₂ représente le radical alkyle en C₁-C₆, où le radical alkyle est substitué une ou plusieurs fois par halogène et phényle, qui à son tour, est substitué une ou plusieurs fois, par halogène, alkyle en C₁-C₆, cycloalkyle en C₃-C₇, un radical carboxyle, un radical carboxyle estérifié par un alcanol en C₁-C₆, un radical trifluorométhyle, un radical hydroxyle, un radical méthoxy, un radical éthoxy, un radical benzyloxy, un radical 2-quinolyle ou un radical 2-, 3- ou 4-pyridyle, où les radicaux 2-quinolyle et 2-, 3- ou 4-pyridyle peuvent être substitués chaque fois, une ou plusieurs fois, par halogène, un radical alkyle en C₁-C₄ ou un radical alcoxy en C₁-C₄,
R₃ et R₄ peuvent être identiques ou différents et représentent hydrogène, alkyle en C₁-C₆, cycloalkyle en C₃-C₇, alcanoyle en C₁-C₆, alcoxy en C₁-C₆, halogène, benzyloxy, un radical nitro, un radical amino, un radical amino mono- ou disubstitué par alkyle en C₁-C₄, un radical (alcoxy en C₁-C₆)carbonylamino et un radical (alcoxy en C₁-C₆)carbonylamino(alkyle en C₁-C₆), et
Z est O ou S.

2. Composé selon la revendication 1, dans lequel R₁ est 4-pyridyle, R₃ et R₄ sont hydrogène, et Z est oxygène.

3. Composé selon la revendication 1 ou 2, où le sel d'addition d'acide est un sel d'un acide minéral ou un sel d'un acide organique.

4. Composé selon la revendication 3, dans lequel l'acide minéral est choisi parmi l'acide chlorhydrique, l'acide sulfurique et l'acide phosphorique, et l'acide organique est choisi parmi l'acide acétique, l'acide lactique, l'acide malonique, l'acide maléique, l'acide fumarique, l'acide gluconique, l'acide glucuronique, l'acide citrique, l'acide embonique, l'acide méthanesulfonique, l'acide trifluoroacétique, l'acide succinique et l'acide 2-hydroxyéthanesulfonique.

5. Composé selon l'une des revendications 1 à 4, où le au moins un indol-3-glyoxylamide N-substitué est combiné à un support, agent de dilution ou auxiliaire utilisable pharmaceutiquement.

6. Composé selon l'une des revendications 1 à 5, où le au moins un indol-3-glyoxylamide N-substitué est formulé sous forme de comprimés, dragées, capsules, solution pour injection, ampoules, suppositoires, emplâtre, composition pulvérulente pouvant être inhalée, suspension, crème ou pommade.

7. Composé selon l'une des revendications 1 à 6, où la tumeur résistant aux médicaments est résistante à au moins un agent anti-tumoral choisi parmi le taxol, la doxirubicine, la vincristine et l'épothilone B.

8. Composé selon l'une des revendications 1 à 7, où le ou les indol-3-glyoxylamides N-substitués sont utilisés sous forme d'une combinaison fixe ou libre avec un agent anti-tumoral connu ou comme substitut d'un agent anti-tumoral qui n'est plus actif par suite de la formation d'une résistance.

9. Composé selon la revendication 8, où l'agent anti-tumoral, qui est utilisé en combinaison avec le ou les indol-3-glyoxylamides N-substitués, est choisi parmi le taxol, la doxirubicine, la vincristine et l'épothilone B.

10. Composé selon la revendication 8, où l'agent anti-tumoral, qui n'est plus actif par suite de la formation d'une résistance, est choisi parmi le taxol, la doxirubicine, la vincristine et l'épothilone B.

11. Composé selon l'une des revendications 1 à 10, où l'indol-3-glyoxylamide N-substitué est choisi parmi le N-(pyridin-4-yl)-[1-(4-fluorobenzyl)indol-3-yl]-glyoxylamide, le N-(pyridin-4-yl)-(1-benzylindol-3-yl)]glyoxylamide, le N-(pyridin-4-yl)-[1-(4-chlorobenzyl)indol-3-yl]glyoxylamide, ou un sel d'addition d'acide physiologiquement compatible de ceux-ci.
